Europäisches Patentamt

European Patent Office

Office européen des brevets

(11) Publication number: **0 563 627 A2**

# EUROPEAN PATENT APPLICATION

(21) Application number: **93103650.3**

(22) Date of filing: **05.03.93**

(51) Int. Cl.5: **A61K 37/02**, A61K 39/395,
A61K 35/14, A61K 35/28,
//C12N15/12,C12P21/08

The applicant has subsequently filed a sequence listing and declared, that it includes no new matter.

(30) Priority: **05.03.92 JP 48840/92**

(43) Date of publication of application:
**06.10.93 Bulletin 93/40**

(84) Designated Contracting States:
**AT BE CH DE DK ES FR GB GR IE IT LI LU MC NL PT SE**

(71) Applicant: **BIO DEFENCE INSTITUTE CO., LTD.**
**Syouei-kannai Building, 22,**
**Sumiyoshi-cho 2-chome,**
**Naka-ku**
**Yokohama-shi, Kanagawa(JP)**

(72) Inventor: **Egawa, Kohji**
**1-11, Kamiyouga 3-chome,**
**Setagaya-ku**
**Tokyo(JP)**
Inventor: **Sato, Ichiei**
**2413, Ishihara-cho**
**Takasaki-shi, Gunma(JP)**

(74) Representative: **Hering, Hartmut, Dipl.-Ing.**
**Patentanwälte**
**Berendt, Leyh & Hering**
**Innere-Wiener-Strasse 20**
**D-81667 München (DE)**

(54) **Anti-tumor method and anti-tumor agent.**

(57) An anti-tumor method and an anti-tumor agent using immunoreaction specific to tumor antigens expressed on the surface of tumor cells. A human non-classical histocompatibility class 1 antigen, an antibody which identify this antigen, or a cytotoxic lymphocyte is administered to a cancer patient as an immunotherapy to induce registance against tumors.

EP 0 563 627 A2

BACKGROUND OF THE INVENTION

1. Field of the Invention

The present invention relates to an anti-tumor method and an anti-tumor agent utilizing immunoreaction specific to tumor antigens produced on the surface of tumor cells.

2. Related art Statement

A technique of producing physiologically active substance having anti-tumor effect from ascites of cancer-bearing animals was disclosed in Japanese Unexamined Patent Laid-Open No. 2-48532.

In that technique, tumor cells are transplanted into the peritoneal cavity of a mammal, an anti-tumor agent is administered, splenocytes obtained from normal syngeneic mammal are administered, and after passing of predetermined period of time, the physiologically active substance was obtained from the ascites pooled in the peritoneal cavity. Otherwise, the physiologically active substance is obtained from supernatant of the ascites collected from the peritoneal cavities of the cancer patients who showed improvement under the immunosurveillance therapy.

However, immunological mechanism to eliminate tumor cells in tumor-bearing animals has not been sufficiently elucidated until now.

An object of the present invention is to provide an anti-tumor method utilizing immunoreaction specific to a tumor antigen.

Another object of the present invention is to provide an anti-tumor agent which utilizes immunoreaction specific to a tumor antigen.

As a method of administering non-classical histocompatibility class 1 antigen, many methods are possible, including:

(1) administering allogeneic lymphocytes which has cross-reactivity with a non-classical histocompatibility class 1 antigen specifically expressed by tumor cells;

(2) administering a non-classical histocompatiblity class 1 antigen produced by a method of genetic engineering; and

(3) introducing isolated non-classical histocompatibility class 1 antigen cDNA into cultured lymphocytes, making the lymphocytes express that non-classical histocompatibility class 1 antigen on the surfaces of the lymphocytes cells, proliferating these lymphocytes in vitro, and administering the lymphocytes thereafter.

These techniques themselves are well known in the art. For example, to produce the antigen in above (2) and (3), a method comprising following steps can be employed:

a. preparing mRNA from tumor cells which express non-classical histocompatibility class 1 antigen;

b. preparing cDNA corresponding to that mRNA using a reverse transcriptase;

c. making cDNA library from the cDNA using λ phage;

d. selecting λ phage which contains objective non-classical histocompatibility class 1 cDNA from the cDNA libraly using a plaque hybridization method;

e. integrating this cDNA into expression vector to produce protein molecule of non-classical histocompatibility class 1 antigen in large quantity using E. coli: and

f. introducing the cDNA obtained in the step d into cultured lymphocytes using a DNA transfection method, culturing these lymphocytes under existence of interleukin 2, and establishing cell clones which express the objective antigen strongly by use of a limiting dilution method.

As a method of preparing an antibody which identifies non-classical histocompatibility class 1 antigen, also many methods known in the art can be employed. For example, a following method can be employed:

1. preparing hybridoma by fusing splenocytes, which are obtained from animals immunized by antigen molecules prepared in the above step e, or lymphocytes, which are obtained from peripheral blood of cancer-bearing individuals and stimulated by culturing in vitro under existence of the antigen molecules obtained in the above step e, with lymphoma cells; and then

2. selecting among these hybridomas ones which produce antibody capable of identifying the objective non-classical histocompatibility class 1 antigen by using a limiting dilution method. For this selection, ELISA using microtiter plates is employed.

Cytotoxic lymphocytes which identifies non-classical histocompatibility class 1 antigen can be prepared also by methods known in the art. For example, a following method can be employed:

1. preparing the objective cytotoxic lymphocytes by culturing splenocytes obtained from animals immunized by the lymphocytes expressing non-classical histocompatibility class 1 antigen, which are

2

prepared In the above step f, or lymphocytes obtained from peripheral blood of tumor-bearing individuals, in vitro under existence of the lymphocytes obtained in the above step f;

2. establishing the objective cytotoxic clones obtained from these lymphocytes using a limiting dilution method; and

3, confirming specificity of identification by measuring cytotoxicity against the lympocytes prepared in the above step f and control lymphocytes which are not introduced with cDNA.

## SUMMARY OF THE INVENTION

The present invention provides an anti-tumor method wherein non-classical histocompatibility class 1 antigen, an antibody which identifies said antigen or a cytotoxic splenocyte is administered, and an anti-tumor agent comprising said antigen as an effective component.

The present invention is described in detail referring to examples in the followings, although the invention is not limited to those examples.

## BRIEF DESCRIPTION OF THE DRAWINGS

Fig.1 is a graph showing existence of Qa-2 antigen on tumor cell strains derived from H-$2^k$ mice;

Fig. 2 is a graph showing results of testing complement-dependent cytotoxic activity of the anti-Qa-2 monoclonal antibody (mAb) and fast-protein-liquid-chromatography (FPLC)-purified material;

Fig. 3 illustrates results of autoradiography of the $^{125}$I-labelled FPLC-purified material;

Fig. 4 is a graph showing adsorption of regressor serum (RS) activity with an anti-IgD-Sepharose column;

Fig. 5 is a graph showing existence of IgD specific to Qa-2 antigen in RS by enzyme-linked immunosorbent assay (ELISA);

Fig. 6 illustrates specificities of oligonucleotides used as primers in polymerase chain reaction (PCR);

Fig. 7 shows a result of DNA-DNA hybridization analysis;

Fig. 8 shows a result of DNA-RNA hybridization analysis;

Fig. 9 shows amplification of BW5147 cDNA by PCR;

Fig. 10 shows reactivity to Qa-2-specific mAb of a fusion protein derived by E. coli; and

Fig. 11 shows nucleotide amplification of cDNAs prepared from human tumor cell strains.

## DETAILED DESCRIPTION OF THE PREFERRED EMBODIMENTS

Example 1:

[Materials and Methods]

(1) Animals

Thirteen syngeneic mouse strains with known Qa, TL and Ly phenotypes (listed in Table 1) were used (KLEIN, J., FIGUEROA, F., DAVID, C.S.; H-2 Haplotypes, gene and antigens: Second listing, Immunogenetics, 1983, 17:553, MCKENZIE, IFC., POTTER, T.; Murine lymphocyte surface antigens, Adv Immunol, 1979, 27:179).

B6, B6.K1 and B6.K2 are congenic with respect to the Qa-1, Qa-2 and Qa-3 antigens (STANTON, TH., BOYSE, EA.; A new serologically defined locus, Qa-1, In the Tla-region of the mouse, Immunogenetics, 1976, 3:525). C3H/He and C3H-Ly6.2 are congenic regarding to allotypes of the Ly6 antigen. The B6.K1, B6.K2 and C3H-Ly6.2 strains were provided by Dr. T. Takahashi of the Aichi Cancer Center Research Institute and maintained by the present inventors.

3

Table 1

| H-2, Qa, TL and Ly phenotypes of mouse strains | | | | | | | | | | | | |
|---|---|---|---|---|---|---|---|---|---|---|---|---|
| Strain | H-2 | Ly -1 | Ly -2 | Ly -3 | Ly -4 | Ly -5 | Ly -6 | Ly -7 | TL | Qa -1 | Qa -2 | Qa -3 |
| C3H/He | k | 1 | 1 | 2 | 1 | 1 | 1 | 2 | b | - | - | - |
| C3H-Ly6.2 | k | 1 | 1 | 2 | 1 | 1 | 2 | 2 | b | - | - | - |
| CBA | k | 1 | 1 | 2 | 1 | 1 | 1 | 2 | b | - | - | - |
| CE | k | 2 | 1 | 2 | 1 | 1 | 1 | 2 | b | - | - | - |
| AKR | k | 2 | 1 | 1 | 1 | 1 | 2 | 2 | b | - | - | - |
| C58 | k | 2 | 1 | 1 | 1 | 1 | 2 | 1 | a | + | - | - |
| BALB/c | d | 2 | 2 | 2 | 1 | 1 | 1 | 2 | c | - | - | - |
| DBA/2 | d | 1 | 1 | 2 | 1 | 1 | 2 | 2 | c | - | + | + |
| DBA/1 | q | 1 | 1 | 2 | 1 | 1 | 1 | 2 | b | | + | - |
| A/J | a | 2 | 2 | 2 | 1 | 1 | 1 | 2 | a | + | + | + |
| C57BL/6 | b | 2 | 2 | 2 | 2 | 1 | 2 | 1 | b | - | + | + |
| B6.K1 | b | 2 | 2 | 2 | 2 | 1 | 2 | 1 | b | + | - | - |
| B6.K2 | b | 2 | 2 | 2 | 2 | 1 | 2 | 1 | b | + | + | - |

(2) Tumor cells

Ascites type cancer cells, MM2, MM46, MM48, MM102D, FM3A, MH134, MH125F, MH125P and X5563 derived from C3H/He mice; ascites type cancer cell, Meth A derived from BALB/c mice; ascites type cancer cell, EL-4 derived from B6 mice; and cultured cancer cell, BW5147 derived from AKR mice; were used.

(3) Monoclonal antibodies and complement

Qa-2-specific monoclonal antibody (mAb) 141-15.8 was purchased from Australian Monoclonal Development, NSW. Sheep antiserum monospecific to each of mouse immunoglobulin subclasses, IgG1, IgG2a, IgG2b, IgG3, IgM and IgA were purchased from Binding Site Ltd., Birmingham, U.K.

Sheep antiserum monospecific to mouse IgD was purchased from ICN ImmunoBiologicals, ILL. Rat mAbs specific to mouse IgG2b and to mouse IgD and peroxidase derivative of the latter were purchased from the Meiji Institute of Health Science, Kanagawa, Japan.

Mouse mAb (IgG2b class) specific to anti-bacterial component (NusA protein of E. coli) was provided by Dr. Y. Nakamura of the Meiji Institute of Health Science and was used as a mAb not related to tumor cell-surface antigens.

F(ab')$_2$ of goat antibody specific to mouse immunoglobulin and its fluorescein isothiocyanate (FITC) derivative were obtained from Tago, Inc., Burlengane, Calif. Fragment Fab' of goat antibody was prepared by reduction of the F(ab')$_2$ with 2-mercaptoethylamine and separation with Sephacryl S-200 column chromatography.

Preabsorbed rabbit serum, used as a complement source, was purchased from Ceder Lane Laboartories, Westbury, N.Y.

(4) Regressor serum (RS)

It was reported that C3H/He mice transplanted with MM2 cancer cells showed tumor regression at a considerable frequency when the cancer cells were removed from the mice together with ascites between the 12th and 14th days after the intraperitoneal inoculation (KIMURA, Y., TANINO-KOMAIJI, T.; Survival of hosts mice and induced resistance to transplantable ascites tumors, Japan J.Exp.Med., 1966 36:371).

Two weeks after removal of the cancer cells, mice which showed complete regression were selected and bled by heart puncture under ether anesthesia. Serum (called in the following as "regressor serum", RS) was separated from the blood and stored at -60°C until used.

(5) Adsorption of RS

For use of RS in adsorption tests, γ-globulins were depleted from RS by precipitation with ammonium sulfate at 0.35 saturation and then by starch gel zone electrophoresis as described below. The resulting material was used as γ-globulin-depleted RS although it may still contain some γ-globulins.

γ-globulin-depleted RS was diluted to 100 times the original volume of RS with RPMI-1640 medium supplemented with 7 % fetal calf serum (FCS). One ml of this solution was mixed with $2 \times 10^7$ lymphocytes (splenocytes or mixture of splenocytes and mesenteric lymphonode cells), which had been freed of erythrocytes by the ammonium chloride method (BOYLE, W.; An extension of cytotoxins, Transplantion 1968, 6:761) and washed three times with Eagle's minimum essential medium (MEM), or with $\overline{(4 \text{ to } 6)} \times 10^6$ ascites tumor cells washed in the same way.

The mixture was incubated at 25°C for 30 min and then at 0°C for 60 min. The cells were removed by centrifugation at 1,000 rpm for 5 min. In case of the adsorption with lymphocytes, the procedure was done twice. The resulting supernatant was used as the adsorbed RS.

(6) Assay of cytotoxicity

Splenocytes of C3H/He mice, obtained 3 days after intraperitoneal transplantation of MM2 cells, were used as the effector cells of the RS-dependent in vitro cytotoxicity reaction (TANINO T, EGAWA K; Regressor serum factor-dependent nonspecific killers in tumor-bearing mice). The effector cells ($1 \times 10^6$) were mixed with $1 \times 10^4$ of $^{51}$Cr-labelled target cells in 0.5 ml RPMI-1640 medium supplemented with 7 % FCS.

In adsorption tests, the γ-globulin-depleted RS before or after the adsorption was added at a final concentration of $500 \times$ dilution of the original RS. MM2 cells, various lymphoblasts and L cell transformants were used as the target cells. The mixture was incubated for 18 h in case of tumor cells, 5 to 7 h in case of lymphoblasts and 10 h in case of L cell transformants at 37°C in a $CO_2$ incubator.

After the incubation, the radioactivity released from the cells into the supernatant and the radioactivity remaining in the cells were determined and the cytotoxicity was expressed in terms of percentage specific lysis. All determinations were carried out in duplicate. In the adsorption tests, results of repeated or different experiments were normalized by expressing them in terms of percentage decrease of the RS activity.

Lymphoblasts were prepared by culturing splenocytes from various mice at a cell density of $1 \times 10^6$ cells/ml for 2 days in RPMI-1640 medium supplemented with 10 % FCS and then for 3 days in the medium containing 5 μg/ml Concanavallin A (Sigma, St. Louis).

$L^{Q7b/Kb}$ and $L^{Kb}$ cells were established by transfection of $Q7^b/K^b$ hybrid gene DNA and $H$-$2K^b$ gene DNA into $L^{tk-}$ cells. $L^{Q7b/Kb}$ cells express the α1 and α2 domains of the $Q7^b$ gene product and α3 domain of $H$-$2K^b$ molecule. The α1 and α2 domains are mainly responsible for immunologically defined Qa-2 specificity of $H$-$2^b$ mouse lymphocytes (WANECK, G.L., SHERMAN, D.H., CLAVIN, S., ALLEN, H., and FLAVELL, R.A.; Tissue-specific expression of cell-surface Qa-2 antigen from a transfected $Qb^7$ gene of C57BL/10 mice, J. Exp. Med., 1987, 165:1358). $L^{Kb}$ cells express the $H$-$2^b$ molecule on their surfaces.

Complement-dependent cytotoxic activity was assayed by the dye exclusion method with mesenteric lymphonode cells as the target cells. The cells ($5 \times 10^5$) were suspended in 100 μl of MEM containing 5 % FCS and the antibody. The suspension was incubated at room temperature for 90 min. The cells were washed twice with MEM, incubated in 100 μl of MEM containing complement at 37°C for 45 min, washed twice with MEM again, added with saline containing 0.2 % trypan blue and examined under the microscope. The percentage lysis was calculated and the value in the absence of antibody was subtracted as the background.

(7) Serum protein fractionation

A serum protein subfraction precipitated between 0.35 and 0.50 saturation of ammonium sulfate at 4°C was fractionated by starch-gel zone electrophoresis using a starch block of $10 \times 40 \times 1.5$ cm buffered with 0.07 M veronal buffer (pH 8.6).

The hydrolyzed starch used for gel electrophoresis was purchased from Connaught Laboratories, Willowdale, Ontario.

After electrophoresis for 24 h at 4°C at 35 mA, the gel was cut into 1-cm-wide blocks and each gel portion was eluted with 10 ml water. The resulting β-globulin fraction was dialysed against 0.01 M TRIS-HCl buffer (pH 8.6), applied on a 1 ml Mono-Q column of fast protein liquid chromatography (FPLC) apparatus (Pharmacia, Uppsala) and was eluted with a 0 to 0.5 M linear gradient of NaCl in the same buffer (TOMONO, T., IKEDA, H., TOKUNAGA, E.; High-performance ion-exchange chromatography of plasma proteins, J Chromatography, 1983, 266:39). After rechromatography, an active fraction with symmetrical elution profile was obtained.

Sepharose 4B conjugated with sheep antisera monospecific to each of mouse IgG1, IgG2a, IgG3, IgA IgM and IgD were prepared using cyanogen bromide-activated Sepharose 4B (Pharmacia, Uppsala) and the respective antibodies. The FPLC-purified material was applied on 0.4 ml columns of each of them at 4°C. The column was washed with saline and eluted with saline containing 5 mM hydrochloric acid. The eluate (0.2 ml fractions) was directly dropped into 0.4 ml RPMI-1640 medium supplemented with 10 % FCS and used for assay of the activity.

(8) Radioiodination of the protein and polyacrylamide gel electrophoresis

The FPLC-purified material (10 $\mu$g) was labelled with $^{125}$I by the chloramine T method (GREENWOOD, HC., HUNTER, WM., GLOVER, JS.; The preparation of $^{131}$I-labelled human growth hormone of high specific radioactivity, Biochem J, 1963, 89:114) using 1 mCi of carrier-free sodium [$^{125}$I]-iodide (NEN). The labelled material was separated from the free iodide by passage through a column of Sephadex G-50.

Two dimensional PAGE (polyacrylamlde gel electrophoresis) was carried out using nonequilibrium pH gradient electrophoresis (NEPHGE) In the first dimension and SDS(sodium dodecyl sulfate)-PAGE after reduction of the material in the second dimension (O'FARRELL, PZ., GOODMAN, HM, O'FARRELL, PH.; High resolution two-dimentional electrophoresis of basic as well as acidic proteins, Cell, 1977, 12:1233).

(9) Detection of Qa-2 antigen by flow cytometry

A T cell-enriched fraction of mouse splenocytes, mouse ascites tumor cells, and BW5147 cells were used. The T cell-enriched fraction was obtained from mouse splenocytes by adsorption to nylon wool (JURIUS, MH., SIMPSON, E., HERZENBERG, LA.; A rapid method for isolation of functional thymus-derived murine lymphocytes, Eur J Immunol, 1973, 3:645). The cells were washed two times with MEM. Cell-surface immunoglobulins of the contaminating B cells were blocked by treating with the Fab' fraction prepared from a goat anti-(mouse immunoglobulin) antibody for 60 min at room temperature.

The cells were then reacted with Qa-2-specific mAb 141-15.8 (200 × dilution). As controls, normal B6.K1 mouse serum or the mAb against a bacterial component were used. The cells were incubated with the mAb for 90 min and then with the FITC(fluorescein isothlocyanate)labelled F(ab')$_2$ fraction of goat antibody specific to mouse immunoglobulin for 60 min at room temperature.

All reagents were diluted in MEM containing 10 % goat serum and 1 % bovine serum albumin (BSA). Incubations were carried out in 50-$\mu$l volumes and the cells were washed two times after each incubation with phosphate buffered saline (PBS) using centrifugation at 5,000 rpm for 2 min in a microcentrifuge. The FITC-labelled cells were fixed with 1 % paraformaldehyde and they were analyzed using a FACSIII apparatus (Becton Dickinson, Mountain View, Calf.).

(10) Detection of serum IgD by enzyme-linked immunosorbent assay (ELISA)

For the detection of Qa-2-specific IgD, Qa-2-lacZ fusion protein, which will be described in Example 2, was used. After inducing the production of the fusion protein in E. coli by isopropylthiogalactoside (IPTG), the cells after the induction were harvested, lysed with 50 % acetic acid. Then, insoluble materials were removed by centrifugation. And, after dialysed extensively against PBS containing 1 % of BSA, the obtained fusion protein was used for enzyme-linked immunosorbent assay (ELISA).

Flat-bottomed 96-well plates (Immunoplate, Nunc) were coated either with the E. coli extract or with sheep anti-(mouse IgD) serum both diluted with 50 mM carbonate buffer (pH 9.0) and then re-coated with 3 % BSA. To each of precoated and washed wells, 50 $\mu$l sample serum, serially diluted in PBS, was added and kept for 2 h at room temperature. The solution was discarded and the wells were washed 3 times with PBS.

Then 50 $\mu$l solution of peroxidase-labelled rat mAb specific to mouse IgD, diluted with 1 % BSA, was added. After standing at room temperature for 1 h, the solution was discarded and the wells were washed 4 times with PBS.

Then, 100 $\mu$l of a mixture of 2,2'-azino-di-(3-ethylbenzthiazoline sulfonate) solution and hydrogen peroxide solution (Kirkegaard and Perry Laboratories, MD) was added to each well and allowed to stand for 5 min at room temperature. The reaction was terminated by adding 100 $\mu$l of 1 % SDS solution. The absorbance at 410 nm of the solution was determined.

[Results]

(1) Recognition specificity of RS factors

It has ben reported that splenocytes, obtained from C3H/He mice bearing ascites tumor cells 3 days after inoculation, lyse various syngeneic (MM2, MM46 etc.) and allogeneic (Meth A, EL-4 etc.) tumor cells by an RS-dependent cellular cytotoxic reaction. Some syngeneic tumor cells (MM48, X5563 etc.) were lysed less strongly by the reaction (TANINO, T., EGAWA, K.; Regressor serum factor-dependent nonspecific killers in tumor-bearing mice).

Such activity of RS was adsorbed to various extents by those tumor cells which were susceptible to the reaction. However, the activity of RS was not adsorbed by those tumor cells which were resistant to the reaction. Susceptibility of the allogeneic tumor cells in general leads to a possibility that the active components in RS (referred as RS factors) recognize allogeneic antigens which might be expressed illegitimately on the tumor cell surface. To test this possibility, RS was adsorbed by lymphocytes obtained from mice of various strains. Results are shown in Table 2.

Table 2

| Adsorption of RS activity with lymphocytes from mice of various strains | | | |
|---|---|---|---|
| Origin of lymphocytes used for adsorption | H-2 haplotype | Target cells | |
| | | MM2 | Meth A |
| | | % Decrease of RS activity* | |
| C3H/He | k | 3.4 ± 2.0 | 6.5 ± 4.3 |
| CBA | k | 2.1 ± 1.8 | 5.5 ± 4.1 |
| CE | k | 23.5 ± 1.4 | 3.6 ± 0.0 |
| AKR | k | 40.5 ± 2.7 | 6.5 ± 6.5 |
| C58 | k | 47.0 ± 2.7 | 24.2 ± 8.2 |
| C57BL/6 | b | 68.7 ± 16.1 | 74.3 ± 12.8 |
| BALB/c | d | 41.8 ± 9.7 | 43.3 ± 3.1 |
| DBA/2 | d | 67.9 ± 11.3 | 79.6 ± 8.7 |
| DBA/1 | q | 46.8 ± 14.8 | 68.9 ± 12.2 |
| A/J | a | 49.8 ± 6.5 | 84.5 ± 9.9 |

* Means ± SE of the results of 2 experiments. Percent specific lysis of MM2 and Meth A cells in the presence of unadsorbed material was 33.2 ± 4.4 and 28.4 ± 4.0, respectively.

As shown in Table 2, the activity of RS, assayed using MM2 target cells, was adsorbed to various extents by lymphocytes with H-2 haplotypes of k (CE, AKR and C58) and those with H-2 haplotypes other than k (B6, BALB/c, DBA/2, DBA/1 and A/J), but not by lymphocytes from C3H/He and CBA mice (both H-$2^k$). Similar but not identical pattern of adsorption was observed when Meth A cells were used as the target cells of the cytotoxicity reaction. The results showed that adsorption of the RS factors occurred when allotypes of one or some of the antiegns in the Ly group of lymphocytes were different from those of C3H/He, or when one or some of Qa/TL antigens were expressed on the splenocytes. Results which further support this were obtained by double-absorption experiments and is shown in Tale 3.

Table 3

| Double adsorption of RS activity with lymphocytes from mice of various strains | | | | |
|---|---|---|---|---|
| Origin of lymphocytes used | | | Target cells | |
| 1st absorption | | 2nd absorption | MM2 | Meth A |
| | | | Decrease of RS activity (%)* | |
| A. | C58 | C3H/He | 37.8 ± 11.5 | 34.4 ± 5.2 |
| | C58 | C57BL/6 | 97.4 ± 2.6 | 97.2 ± 1.7 |
| | C58 | B6.K1 | 33.1 ± 2.2 | 45.1 ± 4.2 |
| | C58 | B6.K2 | 94.1 ± 14.4 | 93.7 ± 4.2 |
| | C58 | DBA/1 | 9.6 ± 6.3 | 94.1 ± 0.3 |
| | C58 | BALB/c | 50.7 ± 6.8 | 38.6 ± 3.1 |
| B. | C57BL/6 | C3H/He | 57.5 ± 4.3 | |
| | C57BL/6 | A/J | 95.7 ± 1.1 | |
| C. | A/J | C3H/He | 38.4 ± 5.6 | 99.3 ± 1.0 |
| | A/J | C57BL/6 | 92.4 ± 0.8 | 103.5 ± 0.7 |
| | A/J | DBA/2 | 96.0 ± 2.8 | 103.9 ± 8.8 |
| | A/J | DBA/1 | 42.8 ± 0.4 | 106.7 ± 1.0 |
| | A/J | BALB/c | 55.2 ± 0.4 | 103.5 ± 2.8 |

* Means ± SE of the results of two experiments. RS preadsorbed doubly with C3H/He lymphocytes was used in the control experiments. The values of percent specific lysis of MM2 and Meth A cells were 27.0 ± 3.0 and 28.2 ± 5.4 respectively in A, 25.4 ± 1.0 in B, and 25.0 ± 1.3 and 28.2 ± 2.0 respectively in C.

A part of the RS activity was adsorbed by C58 lymphocytes. The decrease of the activity after this adsorption suggests that RS contains factor(s) that bind to one or some of Ly1.2, Ly3.1, Ly6.2, Ly7.1, Qa-1 or TL antigens, on the assumption that antigens of Qa/TL or Ly groups are binding targets for RS factors.

Factors that bind to either of Ly2.2, Ly4.2, Ly5.2, Qa-2 or Qa-3, if the serum had any of them, would remain unadsorbed. The remaining activity was adsorbed almost completely by B6, B6.K2 or DBA/1 lymphocytes. Neither B6.K1 nor BALB/c lymphocytes adsorbed the activity factor in the preadsorbed RS significantly. These results suggest that Qa-2 antigen adsorbs some of the activity factors.

Likewise, by adsorption of RS with B6 lymphocytes and then with A/J lymphocytes, the inventors investigated the contributions of Qa-1 and TL antigens to the reaction. Althogh the result was rather ambiguous because of low activity of the preadsorbed RS, it suggested that one or some of Qa-1 and TL antigens may adsorb a part of the activity.

The activity of RS preadsorbed with A/J splenocytes and assayed using MM2 targets was adsorbed almost completely by DBA/2 or B6 lymphocytes but not by DBA/1 or BALB/c lymphocytes. This suggests that the Ly6.2 antigen adsorbs a part of RS activity. When Meth A cells were used as the target, A/J splenocytes adsorbed the activity completely.

Taken altogether, these results suggest that RS contains multiple factors that bind to Qa-2, Ly6.2 and to one or some of the Qa-1 and TL antigens. Phenotypes of all of these antigens are allogeneic to C3H/He. Among them, Qa-1, Qa-2 and TL antigens belong to non-classical histocompatibility class 1 antigen group.

The results also seem to show that MM2 cells express Qa-2, Ly6.2 and one or some of the Qa-2 and TL antigens. Some of the Qa-1, Qa-2 and TL antigens may also be expressed on Meth A cells.

In agreement with the above observations, various allogeneic lymphoblasts which express one or some of these antigens (CE, C58, C57BL/6, B6.K1, B6.K2, DBA/1, DBA/2, A/J and C3H-Ly6.2), but not C3H/He and AKR lymphoblasts, were lysed by the RS-dependent cell-mediated reaction (Table 4). The lysis of C3H-Ly6.2 lymphoblasts but not C3H/He lymphoblasts directly shows the contribution of the Ly6.2 antigen.

The presence of a Qa-2-specific factor was confirmed utilizing such RS-dependent lysis of lymphoblasts of Qa-2,3-congenic mice and of the L cell transformants (Table 5).

The activity of RS to support lysis of B6 and B6.K2 lymphoblasts was adsorbed only partly by B6.K1 lymphocyte but completely by B6 or B6.K2 lymphocytes (Table 5-A). Similar experiments using $L^{Q7b/Kb}$ and $L^{Kb}$ cells show that RS contains an active component with recognition specificity to the $\alpha1/\alpha2$ region of the $Q7^b$ gene product which carries serological Qa-2-specificity (Table 5-B).

Table 4

| RS-dependent cell-mediated lysis of various lymphoblasts | | |
|---|---|---|
| Origin of the lymphoblasts | RS target | |
| | - | + |
| | % Specific lysis* | |
| C3H/He | 0.3 ± 1.2 | 4.2 ± 5.2 |
| C3H-Ly6.2 | 0.0 ± 0.6 | 30.5 ± 12.5 |
| CE | 0.4 ± 0.3 | 20.1 ± 3.5 |
| C58 | 0.2 ± 0.4 | 31.6 ± 5.1 |
| C57BL/6 | 5.2 ± 4.8 | 37.5 ± 12.0 |
| B6.K1 | 0.1 ± 0.2 | 33.2 ± 4.9 |
| B6.K2 | 0.1 ± 0.3 | 37.1 ± 7.0 |
| DBA/1 | 4.8 ± 5.3 | 32.8 ± 2.7 |
| DBA/2 | 2.3 ± 3.2 | 31.4 ± 1.5 |
| A/J | 0.3 ± 0.2 | 36.7 ± 1.2 |

* Means ± SE of the results of two experiments. Ig-depleted RS was added at a final concentration of 500 × dilution.

Table 5

Demonstration of Qa-2-specificity of RS activity by adsorption tests

| Cells used for adsorption | Target cells | |
| --- | --- | --- |
| A. Lymphocytes | Lymphoblasts | |
| | B6.K1 | B6.K2 |
| | Decrease of RS activity(%)* | |
| C3H/He | $0.0 \pm 1.0$ | $-1.9 \pm 11.9$ |
| B6.K1 | $95.8 \pm 8.1$ | $11.6 \pm 17.8$ |
| B6.K2 | $95.1 \pm 3.9$ | $96.9 \pm 1.4$ |
| B. L cell transformants | L cell transformants | |
| | $L^{Q7b/Kb}$ | $L^{Kb}$ |
| % Decrease of RS activity* $L^{Kb}$ | $59.0 \pm 1.6$ | $90.7 \pm 4.5$ |
| $L^{Q7b/Kb}$ | $93.1 \pm 2.4$ | $96.0 \pm 2.2$ |

* Means $\pm$ SE of the results of two experiments. Ig-depleted RS was adsorbed with lymphocytes or L cell transformants and added to the reaction mixture at a final concentration of $500 \times$ dilution. Values of percent specific lysis of B6, B6.K1 and B6.K2 lymphoblasts in the presence of unadsorbed RS were $30.3 \pm 4.2$, $28.3 \pm 1.2$ and $35.4 \pm 2.2$ respectively. Those of $L^{Q7b/Kb}$ and $L^{Kb}$ cells were $45.0 \pm 1.7$ and $33.8 \pm 1.3$ in the presence of unadsorbed RS and $16.3 \pm 2.7$ and $10.1 \pm 1.2$, respectively.

(2) Demonstration of the Qa-2 antigen by flow cytometry

The presence of the Qa-2 antigen on the surface of MM2 and several other tumor cell lines derived from H-2$^k$ (Qa-2$^-$) mice was demonstrated more directly by the membrane immunofluorescence method using a Qa-2-specific mAb 141-15.8.

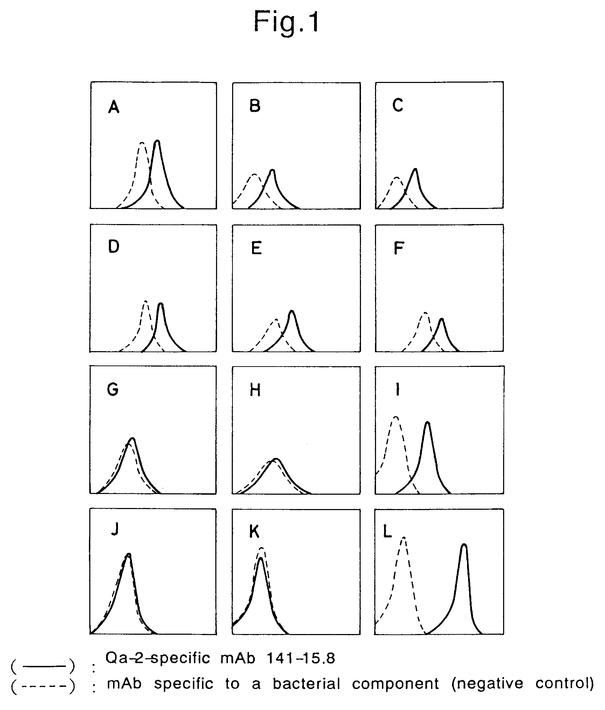

As shown In Fig. 1, significant Qa-2-specific fluorescene was detected reproducibly in seven of the nine cell lines tested (MH134 (A), MH125F (B), MH125P (C), MM2 (D), FM3A (E), MM102D (F), MM46 (G), MM48 (H) and BW5147 (I)). These results show that expression of the Qa-2 allo-antigen on tumor cells is not an event specific to MM2 but is common among various lymphoma and non-lymphoma cell lines and seems to account partly for the wide target selectivity of the RS-dependent reaction.

(3) Characteristics of the Qa-2-specific RS factor

Among multiple factors recognizing allo-antigens presumed to be present in RS, the specificity of a factor to Qa-2 was most clearly demonstrated as described above. The inventors therefore focused their attention on this factor and tried to characterize it further.

By ammonium sulfate precipitation and preparative electrophoresis of the serum protein, the activity to support the cell-mediated lysis of B6 lymphoblasts, but not of B6.K1 lymphoblasts, was found mainly in the $\beta$-globulin fraction. This fraction was then fractionated by FPLC using the Mono-Q column.

The activity was associated with the material eluted at 0.30 M NaCl, whereas almost all of the mouse mAbs of the IgG subclasses and those of the IgM class were eluted at around 0.25 M and 0.35M NaCl positions, respectively.

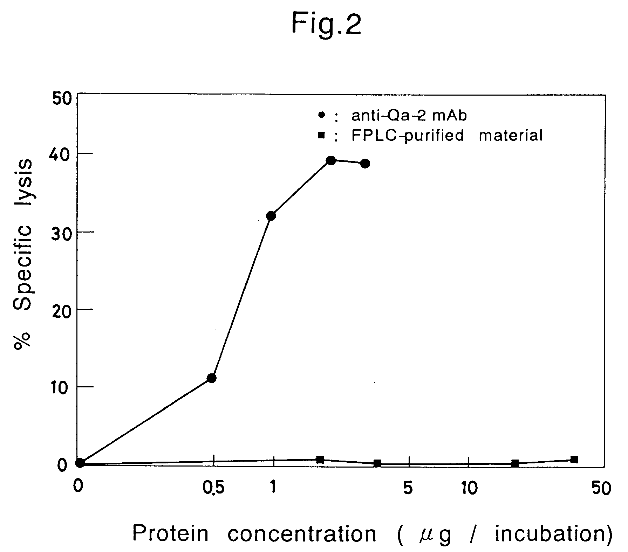

The semi-purified material was also tested for complement-dependent cytotoxic activity against Qa-2$^+$ lymphocytes (Fig. 2). Complement-dependent lysis of B6 mesenteric lymphnode cells by the FPLC-purified material was not detected even when 35 $\mu$g protein was used per assay. On the other hand, anti-Qa-2 mAb of the IgG2b class in the presence of complement lysed the same target cells at an antibody concentration as low as less than 1 $\mu$g protein per assay.

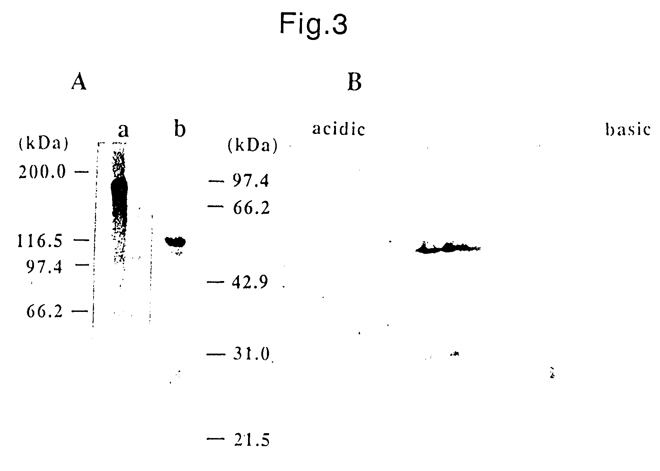

To further characterize the Qa-2-specific RS component, the FPLC-purified material was radiolabelled with $^{125}$I, preadsorbed with C58 lymphocytes, adsorbed to the surface of Qa-2$^+$ cells and analysed by SDS-PAGE or by two dimensional PAGE followed by autoradiography (Fig. 3).

In Fig. 3, A shows one-dimensional SDS-PAGE under non-reducing (a) and reducing (b) conditions, using a 7.5 % polyacrylamide gel; and B shows pattern of two-dimensional PAGE.

Specific binding of about 2 % of the total radioactivity to the cells was observed. The relative mass of the bound material under non-reducing condition was estimated to be 160 kDa. Under reducing conditions, it showed bands with relative masses of appoximately 50 kDa and 25 kDa. These results revealed that the material had a heavy and light chain structure similar to that of IgG. The two dimensional gel electrophoresis of the labelled material showed that it was composed of four or more components whose isoelectric points (PI) ranged approximately from 5.5 to 6.5 and all of which had heavy and light chain structures.

It is known that IgD are found in the $\beta$-globulin fraction of serum proteins and comprise a group of acidic glycoproteins (ISHIHARA, E., TEJIMA, Y. TAKAHASHI, R. TAKAYASU, T., SHINODA, T.; Structure and location of a sparagine-linked olygosaccharides in the Fc region of a human immunoglobulin D, Biochem Biophys Res Comm, 1983, 110:181), whereas IgGs in general are basic proteins. IgD has a structure composed of heavy and light chains like IgGs, but does not interact with C1q of the complement system (SPIEGELBERG, HL.; Immunoglobulin D (IgD), Methods in Enzymology, 1985, 116:95, SPIEGELBERG, HL.; The structure and biology of human IgD, Immunological Rev, 1977, 37:3).

It has also been reported that there are two forms of mouse serum IgD. One has a molecular mass of about 170-200 kDa, while the other, which lacks the C1 domain of the heavy chains, has a molecular mass of approximately 150-160 kDa (FINKELMAN FD., KESSLER, SW., MUSHINSKI, JF., POTTER, M.; IgD-secreting murine plasmacytomas: Identification and partial characterization of two IgD myeloma proteins, J Immunol, 1981, 126:680, MOUNTS, JD., MUSHINSKI, JF., OWENS, JD, FINKELMAN, FD.; The in vivo generation of murine IgD-secreting cells is accompanied by deletion of C $\mu$ gene and occasional deletion of the gene for the C$\delta$1 domain, J Immunol, 1990, 145:1583). Variety in the sugar moieties of a human monoclonal IgD (NIG-65 myeloma protein) seems to cause heterogeniety in the isoelectric point between 5.6 and 6.8.

These characteristics agree with those of the serum component with Qa-2 specifity. For this reason, the inventors speculated that the Qa-2 specific component is an IgD.

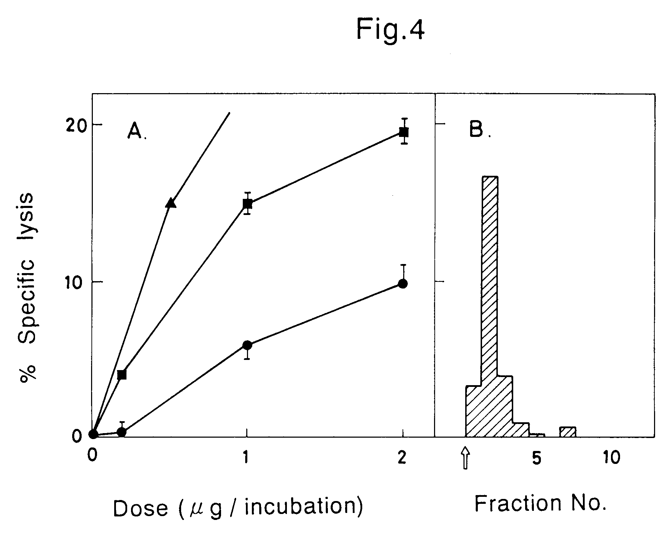

To test this possibility, the FPLC-purified material after adsorption with L$^{kb}$ cells was further adsorbed with Sepharose 4B conjugated with each one of the antibodies specific to various mouse immunoglobulin subclasses. In Fig. 4-A, are shown the cell-mediated lytic activity to Q$^{7b/Kb}$ cells of the material before the adsorption (▲), after adsorption with L$^{Kb}$ (■) and after further adsorption with the Sepharose 4B (●). The activity demonstrated by using L$^{Q7b/Kb}$ as the target cells was adsorbed considerably with Sepharose 4B conjugated with anti-IgD.

The material retained on and eluted from the anti-IgD column had significant activity to support lysis of L$^{Q7b/Kb}$ cells (Fig. 4-B). The arrow in Fig. 4-B shows the position of the start of the elution.

The activity was not significantly adsorbed with Sepharose 4B conjugated with either one of anti-IgG1, anti-IgG2b, anti-IgG3, anti-IgA and anti-IgM. Only slight activity was adsorbed to and recovered from an anti-IgG2a-conjugated Sepharose 4B column. These results were reproducible and shows that the active component in RS with Qa-2-specifity was an IgD.

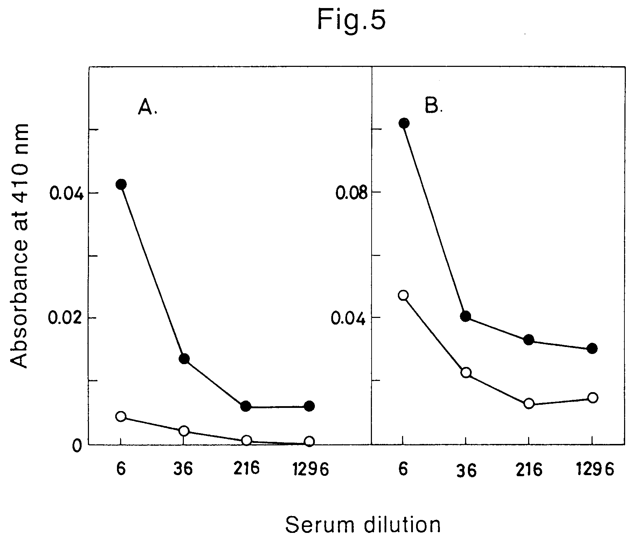

Presence of IgD in RS with specificity to Qa-2 antigen was further demonstrated by ELISA using the Qa-2-lacZ fusion protein. As shown in Fig. 5, Qa-2-specific IgD was detected in RS whereas it was not detected in the serum of normal adult C3H/He. In accordance with the appearance of the Qa-2-specific IgD, an increase of total amount of serum IgD was also detected in RS compared to the normal control serum.

Minor amounts of Qa-2-specific IgG2a and IgG2b were also detected in RS by ELISA using the fusion protein-coated plates and peroxide-labelled rat mAbs specific to mouse IgG2a and IgG2b, respectively. Qa-2-specific IgG2a and IgG2b were not detected in the FPLC-purified material while Qa-2-specific IgD was detected in it.

From these results, it is concluded that the serum component with Qa-2-specific activity in RS is an IgD.

Example 2:

[Materials and methods]

(1) Mice

C3H/He, AKR, B6 and B6.K1 mice were used. The former 2 mice have H-$2^k$ and Qa-$2^-$,$3^-$ phenotypes. B6 and B6.K1 mice have phenotypes of Qa-$1^-$,$2^+$,$3^+$,and of Qa-$1^+$,$2^-$,$3^-$, respectively.

(2) Tumor Cells.

BW5147 is an in vitro cell line derived from a T cell lymphoma of the AKR mouse. MM2 and FM3A are ascites cell lines of virally induced mammary carcinomas of C3H/He mouse. MH134 is an ascites cell line of a chemically induced hepatoma of C3H/He.

It has been shown that these cell lines reacted with a Qa-2-specific mAb 141-15.8 (TANINO, T., SEO, N., OKAZAKI, T., NAKANISHI-ITO, C., SEKIMATA, M. and EGAWA, K.; Humoral responses to Qa-2 and other tumor antigens in mice, submitted for publication in Cancer Immunology Immunotheraphy).

$L^{Q7b/Kb}$ and $L^{Kb}$ cells were established by transfection of $Q7^b/K^b$ hybrid gene DNA and of H-$2K^b$ genomic DNA, respectively. $L^{Q7b/Kb}$ cells express $\alpha1$ and $\alpha2$ domains of the $Q7^b$ gene product and $\alpha3$, transmembrane and cytoplasmic domains of the H-$2K^b$. $L^{Kb}$ cells express H-$2K^b$ antigen. Both cells as well as non-transfected L cells express H-$2^K$ antigens.

(3) Antibodies

MAb 141-15.8, which has specificity to the Qa-2 antigen, was obtained from the American Type Culture Collection, MA.

MAb 34-1.2, which has specificity to Qa-2 antigen, was obtained from Australian Monoclonal Development, NSW.

A hybridoma clone producing Qa-2-specific mAb 59 was newly established by fusing NS-1 cells with lymphocytes from B6.K1 immunized with B6 lymphocytes.

Thy1.1-specific mAb was purchase from Meiji Institute of Health Science, Japan. Thy1.2-specific mAb was purchaced from Ceder Lane Laboratories, N.Y.

A mouse mAb of the IgG2b class specific to a bacterial component (NusA protein) was kindly provided by Dr. Y. Nakamura of Meiji Institute of Health Science and used as a mAb not related to tumor cell surface antigens.

F(ab')$_2$ of goat antibody specific to mouse immunoglobulin and its FITC derivative were obtained from Tago Inc., CA. Fab' fragment of the antibody was obtained by reducing the F(ab')$_2$ with 2-mercaptoethylamine and fractionating by Sephacryl S-200 column chromatography.

(4) Treatment of tumor cells with phosphatidylinositol-specific phospholipase C (PI-PLC)

PI-PLC1 derived from Bacillus thurigiensis (IKEZAWA, H., and TAGUCHI, R.; Phosphatidylinositol-specific phospholipase C from Bacillus cereus and Bacillus thurigiensis, Method in Enzymol. 1981, 71:731) was purchased from Sapporo Beer Co., Tokyo.

Cancer cells ($2 \times 10^6$) were incubated at 37°C for 60 min in 30 $\mu$l of D-MEM (pH 7.5) containing 0.1 mU of PI-PLC and 1 mg/ml of ovalbumin. After the incubation, the cells were washed twice with D-MEM and used for detection of cell surface antigens.

### (5) Immunofluorecence analysis

Detection of cell surface Qa-2 and Thy1 antigens was carried out by flow cytometry. Briefly, the cells were reacted with Qa-2-specific mAb and then with the FITC-labelled F(ab')$_2$ fraction of goat antibody specific to mouse immunoglobulins.

In the cases of lymphocytes, T cells were enriched by passing through a Nylon wool column and cell surface immunoglobulins of contaminating B cells were blocked by treating with Fab' fragment of the antibody. The FITC-labelled cells were fixed with 1 % paraformaldehyde and analyzed using the fluorescence activated cell sorter (FACS III, Becton Dickinson, CA).

### (6) Preparation of cDNA

Total cellular RNA was extracted from tumor cells and thymocytes by the guanidine isothiocyanate method (SCHIBLER, U., TOSI, M, PINETT, A.-C., FABIANI, L., and WELLAUER, P.K.; Tissue-specific expression of mouse alpha-amirase genes, J Mol Biol, 1980, 142:93), and was enriched for mRNA by isolating poly(A)$^+$ RNA on oligo(dT)cellulose (Boehringer Mannheim GmbH, Germany) (AVIV, H., and LEDER, P.; Pulification of biologically active globin messenger RNA by chromatography on oligothymidylic acid cellulose, Proc Natl Acad Sci USA, 1972, 69: 1408). Synthesis of cDNA from the mRNA was carried out using a cDNA synthesis kit (cDNA plus, Amersham Corp., Arlington Heights, IL) with oligo-dT as a primer.

### (7) Oligonucleotlde primers and probes

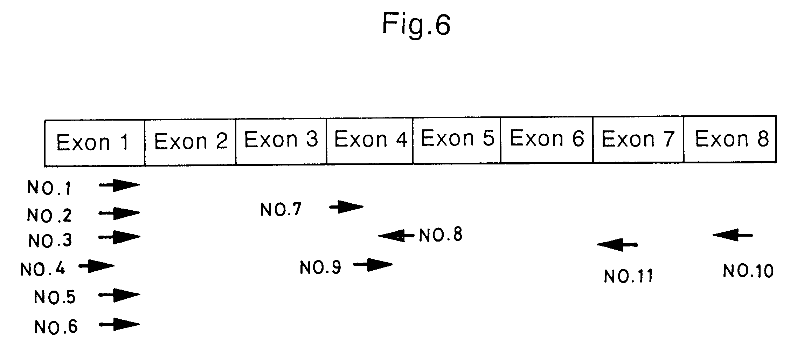

Based on the reported nucleotide sequences of genes in the Qa-2, 3 region of C3H/He (WATTS, S., DAVIS, A.C., GANT, B. WHEELER, C., HILL, L. and GOODENOW, R.S.; Organization and structure of the Qa genes of the major histocompatibility complex of the C3H mouse, Implications for Qa function and class I evolution, EMBO J, 1989, 8:1749), eleven 20mer oligonucleotides were prepared on a DNA synthesizer (Applied Biosystems model 391) and used as primers for polymerase chain reaction (PCR). Their sequences and specificites are shown in Table 6 and Fig. 6.

Oligonucleotides Nos. 1, 2, 3, 4, 5 and 6 were complementary to specific antisense sequences found in the first exons of each of the H-2D$^K$, Q1$^K$, Q2$^K$, Q4$^K$, Q5$^K$ and Q10$^K$ genes, respectively. Nos.10 and 11 were complementary to a Q5$^K$-specific sense sequence in exon 8 and exon 7, respectively. Nos. 7, 8 and 9 were complementary to sequences common to all class 1 genes. Oligonucleotides Nos. 12 to 17, complementary to Nos. 1 to 6 respectively, were also synthesized and used as probes in DNA-RNA hybridization experiments.

Table 6

| Symthetic deoxyoligonucleotides used as primers and proves | | |
|---|---|---|
| Name | Sequence(5'-3') | Specificity |
| No.1 | TGGCCCCGGCTCAGACCCGC | H-2D$^K$ |
| No.2 | TGACCCTGACCAAAACCGGA | Q1$^K$ |
| No.3 | CCCGGACCCAGAACCGAGCC | Q2$^K$ |
| No.4 | AGTCGCCCAGACCCTGATCG | Q4$^K$ |
| No.5 | CTCTGACCCAGACCCGCGCT | Q5$^K$ |
| No.6 | CCCCGACCCAGACCCAGGCA | Q10$^K$ |
| No.7 | GCTGGGCCCTGGGCTTCTAC | class 1 |
| No.8 | AGGGTGAGGGGCTCAGGCAG | class 1 |
| No.9 | CTGGCAGTTGAATGGGGAGG | class 1 |
| No.10 | ATCGTCTGTCACTCAGTCCA | Q5$^K$ |
| No.11 | GCTCTAGGAGCTGTCCCTGC | Q5$^K$ |
| No.12 to No. 17 | Complementary to No.1 to No.6, respectively | |

(8) Polymerase chain reaction (PCR)

PCR was carried out in 100 $\mu$l reaction mixture containing 50 ng of DNA, 50 pmoles each of primers complementary to sense and antisense sequences, 20 mM Tris-chloride (pH 8.3), 1.5 mM MgCl$_2$, 25 mM KCl, 0.05 % Tween 20, 0.1 mg/ml gelatin, 50 $\mu$M each of dNTP and 2 units of Taq DNA polymerase (Parkin-Elmer/Cetus, Norwalk, CT). The reaction mixture was overlayed with 100 $\mu$l of mineral oil to prevent evaporation. The thermal cycler (Atto, Tokyo, Japan) was run 40 cycles (each of 1 min at 94°C, 1.8 min at 55°C and 2 min at 72°C).

(9) Cloning and sequencing of PCR-amplified DNA

DNA amplified by PCR was sequenced either directly or after cloning into the SmaI site of pUC119. For the cloning, competent E. coli MV1184 cells were transformed with the plasmid DNA and grown on a plate containing X-gal, IPTG and ampicillin. White colonies were picked and grown in 2xYT broth. The cells were then infected with M13K07 helper phage.

The resulting single strand DNA, as well as the PCR-amplified DNA, was sequenced by the dideoxy method (SAMAGE, F., NICKLEN, S. and COULSON, A.R.; DNA sequencing with chain-terminating inhibitors, Proc Natl Acad Sci USA, 1977, 74:5463) using a DNA sequecing kit (Sequenase version 2.0, United States Biochemical Corp., Cleaveland, OH) and synthetic oligonucleotides used as PCR primers or M13 universal primer.

(10) Preparation of genomic DNA and DNA-DNA hybridization

Splenocytes from B6, C3H/He and AKR mice and BW5147 cells were incubated at 60°C overnight in 0.03 % SDS solution containing 0.1 mg/ml proteinase K and then extracted with phenol. The resulting aqueous phase was dialyzed overnight against 10 mM Tris-chloride (pH 7.5) containing 1 mM EDTA and used as the DNA solution.

Ten $\mu$g each genomic DNA was digested with HindIII or with BamHI, electrophoresed through 0.8 % agarose gel and transferred onto a Nylon filter (Hybridon-N$^+$, Amersham). The filter was dried at 37°C and incubated for 1 h at 60°C in 6 ml of hybridization buffer (6 × SSC, 5 × Denhardt's solution, 0.5 % SDS, 50 % formamide, 20 mM sodium phosphate buffer, pH 6.5) containing 1 mg of salmon sperm DNA. The DNA probe which had been labelled with $^{32}$P by the random primer method was then added to the incubation. Hybridization was carried out at 42°C overnight.

The filter after the hybridization was washed twice at room temperature for 15 min in 2 × SSC containing 0.1 % SDS and twice at 37°C for 30 min in 0.2 × SSC containing 0.1 % SDS. The filter was then rinsed with 0.1 × SSC and exposed to X-ray film using an intensifier screen.

14

(11) DNA-RNA hybridization

Poly(A)$^+$ RNA was prepared as described above from AKR thymocytes and BW5147 cells, denatured with glyoxal, electrophoresed through 1.2 % agarose gel containing 20 mM phosphate buffer (pH 7.4) and transferred onto a Nylon filter. The filter was dried at 37°C and incubated for 1 h at 60°C in 2 ml of the hybridization buffer containing 100 $\mu$g salmon sperm DNA.

Oligonucleotide probes, 5'-terminus of which had been labelled with $^{32}$P, was then added to the incubation. After incubating at 42°C overnight, the filter was washed twice with 2 × SSC for 15 min at room temperature and twice with 2 × SSC containing 0.1 % SDS for 30 min at 50°C. The filter was then rinsed with 0.2 × SSC and exposed to X-ray film using an intensifier screen.

(12) Synthesis of Q5$^K$ protein and Western blotting.

DNA corresponding to exons 1 to 4 of the Q5$^K$ gene which had been amplified from BW5147 cDNA was cloned into pUC119 and sequenced as described above. A colony was selected which had the insertion in the right direction and in frame downstream of the lacZ promotoer. This colony was grown in L both containing Ampicillin until the optical density at 600 nm reached 0.3. IPTG was added to the culture to 50 $\mu$M and culturing continued until the optical density became 0.75.

The cells were harvested, precipitated with 10 % trifluoroacetic acid, washed with acetone and dissolved by heating for 30 min at 100°C in 25 mM Tris-hydrochloride (pH 6.8) containing 2 % SDS, 5 % glycerol, 0.0125 % BPB and 2.5 % 2-mercaptoethanol.

The dissolved material was electrophoresed through a 12 % polyacrylamide gel containing 0.1 % SDS and transferred onto polyvinyl difluoride membrane (Immobilon, Millipore, Bedford, NA) by electroblotting in a solution composed of 25 mM Tris base, 192 mM glycine, 20 % methanol and 0.02 % SDS. The membrane was then rinsed with 0.15 M sodium chloride containing 0.2 % Tween 20 and buffered with 10 mM Tris-hydrochloride (pH 7.6) (TTBS), incubated for 2 h at 37°C in TTBS containing 10 % goat serum and 10 % horse serum.

The membrane was rinsed with water and incubated overnight at room temperature in 200 × diluted mouse ascites containing mAb 59 in 5 ml of the same medium. After the incubation, the filter was rinsed 3 times with TTBS and reacted for 1 h at 37°C with peroxidase-labelled anti-mouse IgG (Amersham) in saline buffered with 10 mM Tris-borate (pH 7.6). The membrane was again washed 3 times with TTBS and treated for coloration in 50 ml of TTBS containing 40 mg of 3,3'-diaminobenzidine tetrahydrochloride (Wako Chemicals, Osaka, Japan) and 15 $\mu$l of 30 % hydrogen peroxide.

[Results]

(1) Characteristic of the Qa-2 antigen expressed on tumor cells derived from H-2$^K$ mice.

In the report of TANINO, T., SEO, N., OKAZAKI, T., NAKANISHI-ITO, C., SEKIMATA, M., and EGAWA, K; Humoral responses to Qa-2 and other tumor antigens in mice, (Submitted for publication in Immunogenetics), it was demonstrated that most tumor cell lines (7 out of 9 cell lines tested) derived from $\overline{\text{H-}2^K \text{ (Qa-2}^-)}$ mice express the Qa-2 antigen detected by the membrane immunofluorescence method using anti-Qa-2 antiserum or Qa-2-specific mAb 141-15.8.

It was found, however, that the Qa-2 antigen was not detected on these cells in general when another Qa-2-specific mAb 34-1.2 was used. Qa-2-specificity of the newly obtained mAb 59 was examined and compared to those of mAbs 141-15.8 and 34-1.2. The result is shown in Table 7.

Table 7

| Detection of Qa-2 antigen on various cells using anti-Qa-2 monoclonal antibodies | | | | |
|---|---|---|---|---|
| Cell | Qa-2-specific mAb | | | Non-related mAb |
| | 34-1.2 | 59 | 141-15.8 | |
| | Mean fluorescence intensity | | | |
| B6 lymphocyte | | 156.1 | 59.0 | 22.3 |
| B6.K1 lymphocyte | | 30.7 | 25.0 | 30.9 |
| C3H/He thymocyte | 16.7 | 17.4 | 16.6 | 16.8 |
| AKR thymocyte | 18.1 | 19.2 | 19.5 | 18.0 |
| MM2 | 58.9 | 101.3 | 102.8 | 64.8 |
| BW5147 | 46.4 | 80.4 | 102.3 | 45.2 |
| L$^{Q7b/Kb}$ | 192.0 | 180.2 | 42.1 | 48.4 |
| L$^{Kb}$ | | 81.2 | 68.2 | 71.5 |

It is evident that mAb 59 recognizes the $\alpha 1$ or $\alpha 2$ domain of the Qa-2 molecule which is the product of the Q7$^b$ gene and does not cross-react with at least the H-2$^k$ and H-2$^b$ antigens. Unlike mAb 34-1.2, mAb 59 reacted not only with H-2$^b$ lymphocytes but also with various H-2$^k$ tumor cells such as MM2 and BW5147.

Such reactivity to Qa-2-specific mAbs indicates that these tumor cells express a Qa-2 antigen which is closely related to but not identical to the Qa-2 antigen detected on normal lymphocytes of H-2$^b$ mice and that the $\alpha 1$ or $\alpha 2$ domain of the molecule is responsible for at least a part of the difference of these two Qa-2 antigens. Table 7 also shows that Qa-2 antigen was not detected on thymocytes and splenocytes of H-2$^k$ mice (C3H/He and AKR) when either one of these mAbs was used.

It has been reported that the Qa-2 antigen expressed on lymphocytes obtained from H-2$^b$ mice is susceptible to digestion with PI-PLC (SCHIBLER, U., TOSHI, M., PINETT, A.-C., FABIANI, L., and WELLAUER, P.K.; Tissue-specific expression of mouse alpha-amirase genes, J Mol Biol, 1980, 142:93), showing that the molecule is anchored to the cell membrane via a glycophosphatidylinositol (GPI) moiety.

It is known that the GPI structure is linked to an aspartic acid of the cell surface protein molecule (LOW, M.G.; Biochemistry of the glycosyl-phosphatidyl inositol membrane anchors, Biochem J, 1987, 244:1). With regard to the Qa-2 molecule, the GPI anchor has been supposed to be linked to an aspartic acid residue at position 295 coded by a CAC in exon 5 of Q7$^b$ gene (WANECK, G.L., SHERMAN, D.H., KINCADE, P.W., LOW, M.G. and FAVELL, R.A.; Molecular mapping of signals in the Qa-2 antigen required for attachment of the phosphatidylinositol membrane anchor, Proc Natl Acad Sci USA, 1988, 85:577). In H-2 antigen molecules, which are resistant to treatment with PI-PLC, this aspartic acid residue is replaced by valine.

DNA sequences of genes of the Qa-2,3 region reported so far show that all putative Q gene products other than Q7 and Q9 have valine at this position (DEVLIN, J.J., WEISS, E.H., PANSLON, M. and FLAVELL, R.A.; Duplication of gene pairs and alleles of class I genes in the Qa-2 region of the murine major histocompatibility complex: a comparison, EMOB J, 1985, 4:3203).

From these observations, it was supposed that the Qa-2 antigen expressed on H-2$^k$ tumor cell surfaces might be resistant to PI-PLC treatment. As shown in Table 8-A, Qa-2 molecules detected by mAb 141-15.8 on various tumor cells derived from H-2$^k$ mice were resistant to PI-PLC treatment.

In a control experiment, susceptibility of the Thy-1 antigen on BW5147 cells was tested. The Thy-1 antigen is also known to be one of the cell surface molecules having a GPI anchor (LOW, M.G., and KINDADE, P.W.; Phosphatidylinositol is the membrane-anchoring domain of the thy-1 glycoprotein, Nature, 1985, 318:62).

As shown in Table 8-B, the Thy-1 antigen expressed on BW5147 cells was susceptible to PI-PLC treatment. This result showed that the lack of a GPI anchor on the Qa-2 antigen of BW5147 cells was not due to a defect in the cellular mechanism to synthesize GPI-anchors, but due to biochemical differences of the Qa-2 molecules themselves.

Table 8

Susceptibility of Qa-2 and Thy1 antigens to PI-PLC treatment

A. Antibody

| | mAb 141-15.8 | | non-related mAb | |
|---|---|---|---|---|
| | PI-PLC treatment | | | |
| Cell | - | + | - | + |
| | Mean fluorescence intensity | | | |
| B6 lymphocyte | 152.0 | 59.7 | 30.6 | 29.4 |
| BW5147 | 85.1 | 96.4 | 35.8 | 36.2 |
| MM2 | 88.2 | 98.0 | 73.2 | 74.6 |
| FM3A | 108.0 | 109.3 | 71.1 | 72.3 |
| MH134 | 101.2 | 102.9 | 67.9 | 72.5 |

B. Antibody

| | anti-Thy1.1 | | anti-Thy1.2 | | non-related mAb |
|---|---|---|---|---|---|
| | PI-PLC treatment | | | | |
| Cell | - | + | - | + | - |
| | Mean fluorescence intensity | | | | |
| AKR thymocyte | 193.0 | 98.0 | | | 34.5 |
| B6 thymocyte | | | 176.0 | 79.7 | 40.1 |
| BW5147 | 176.0 | 95.1 | | | |

From these obsevatoins, it is evident that the Qa-2 antigen expressed on tumor cells derived from $H-2^k$ mice (Qa-$2^k$ antigen) is distinct immunologically and biochemically from that expressed on $H-2^b$ normal lymphocytes. The Qa-$2^k$ antigen can be defined as a PI-PLC-resistant molecule expressed on tumor cells derived from $H-2^k$ mice and which is reactive to mAbs 59 and 141-15.8 but not to mAb 34-1.2.

(2) Identification of the gene encoding the Qa-$2^k$ phenotype of BW5147 cells

Among the Qa-$2^k$-positive tumor cells, BW5147 cells grown in vitro were selected for further tests because expression of Qa-$2^k$ was fairly strong and also because the cells could be obtained without contamination of cells from the host animals.

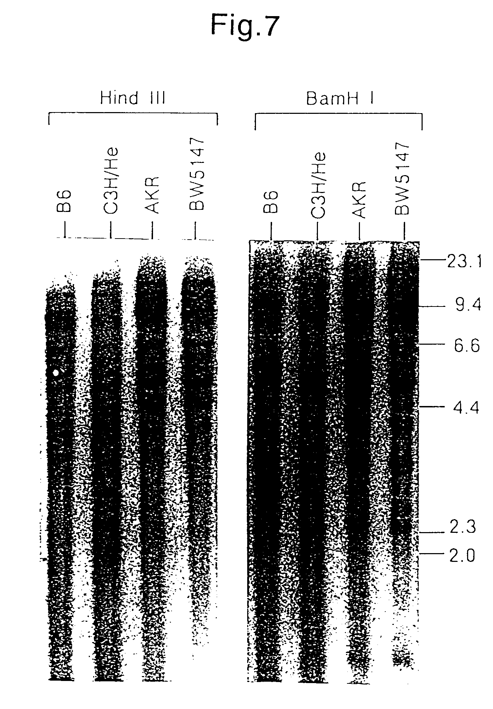

Fig. 7 shows the results of DNA-DNA hybridization analyses in which HindIII or BamHI fragments of genomic DNAs were hybridized with a $^{32}$P-labelled DNA probe of about 800 bp which could detect the DNA region containing exons 2, 3 and 4 of all class 1 genes.

The patterns of autoradiograms obtained using DNAs from C3H/He mice, AKR mice and BW5147 cells were essentially identical and they were distinct from that obtained using B6-mouse DNA. From these results, it is considered that the arrangement of class 1 genes was conserved among genomes of these H-$2^k$ mice (C3H/He and AKR) and the lymphoma cell line derived from AKR.

It was reported that the Qa-2,3 region of the C3H/He genome contained $Q1^k$, $Q2^k$, $Q4^k$, $Q5^k$ and $Q10^k$ genes (WATTS, S., DAVIS, A.C., GANT, B., WHEELER, C., HILL, L., and GOODENOW, R.S.; Organization and structure of the Qa genes of the major histocompatibility complex of the C3H mouse. Implications for Qa function and class I evolution, EMBO J, 1989, 8:1749, WEISS, E.H., BEVEC, D., MESSER, G., SCHWEMMLE, S., GROSSHAUSE, C., STEINMETZ, M. and SCHMIDT; Organization of the AKR Qa region: Structure of a divergent class I sequence, $Q5^k$, J Immunogenet, 1989, 16:283).

The $Q3^k$ gene was a pseudogene and genes corresponding to Q6, Q7, Q8 and a large part of Q9 were deleted. The $Q5^k$ gene seemed to be a hybrid gene composed of a large 5' part of the Q5 gene and a short 3' part of the Q9 gene.

The inventors tried to identify the gene which was silent in AKR and transcribed in BW5147 by DNA-RNA hybridization and also by amplification of cDNA by PCR using primers and probes specific for each of the $Q1^k$, $Q2^k$, $Q4^k$, $Q5^k$ and $Q10^k$ genes (Table 6 and Fig.6).

A primer and probe specific to H-$2D^k$ gene was also used as a positive control.

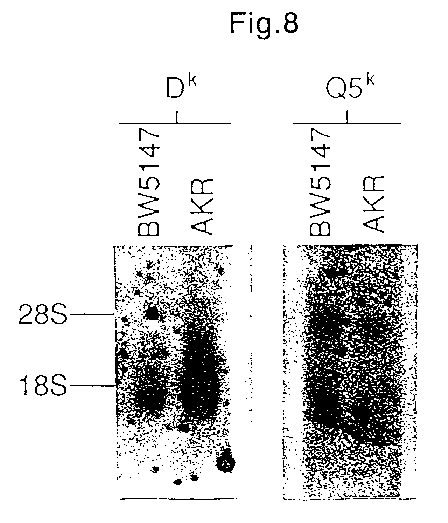

Fig. 8 shows the results of DNA-RNA hybridization test. H-$2D^k$-specific probe (No.12) showed a specific hybridizing band at 1.5 kb with both AKR thymocyte RNA and BW5147 RNA. In contrast, $Q5^k$-specific probe (No.16) hybridized to approximately 1.2, 1.5 and 4.0 kb species in BW5147 RNA, whereas no hybridization was detected with regard to AKR RNA.

Also, hybridization either to AKR RNA or BW5147 RNA was not detected when probes No. 13, 14, 15 and 17 were used (data not shown). These results indicated that $Q5^k$ gene, the transcription of which was not detected in H-$2^k$ thymocytes, was specifically transcribed among Q genes in BW5147 cells. The transcription of $Q5^k$ gene in BW5147 cells was supported by the results of PCR.

Using primers specific to H-$2D^k$, $Q1^k$, $Q2^k$, $Q4^k$, $Q5^k$ and $Q10^k$ (No.1 to No.6, respectively) and a primer (No. 8) which was complementary to a class 1 common sense sequence in the 3'-terminal region of exon 4, the PCR was carried out.

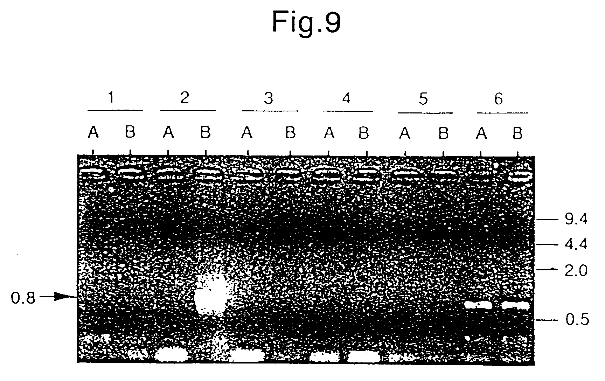

As shown in Fig. 9, amplification of BW5147 cDNA was successful only when primers No.1 and No.5, which were complementary to H-$2D^k$ and $Q5^k$ exon 1 antisense sequences, respectively, were used together with primer No.8. Amplification was not detected when primers No. 2, 3, 4 and 6 were used together with primer No.8 even when the anealing temperature was lowered to 45 °C. The DNA amplified using priemrs No.5 and No.8 was sequenced, directly and also after cloning into pUC119, using primers No.5, 7, 8 and M13 universal primer.

The sequence was found to be identical to the reported nucleotide sequence of exons 1 to 4 of the $Q5^k$ gene of C3H/He. Amplification of genes other than Q5k was not detected during direct sequencing. Amplification of the H-$2D^k$ sequence from AKR cDNA was also detected using primers No.1 and No.8.

However, none of the Q-specific sequence could prime amplification of the AKR cDNA. Amplification of BW5147 cDNA was observed also when primer No.9, which was complementary to a class 1 common antisense sequence in exon 4, and primer No.10, which was complementary to a $Q5^k$-specific sense sequence in exon 8, were used. The nucleotide sequence of the DNA amplified using these primers was determined directly using primers No.9 and 10.

Nucleotide sequence of cDNA, deduced from these results, coincided completely with the nucleotide sequence prospected from the reported sequence of $Q5^k$ gene. It, therefore, is evident that, $Q5^k$ gene, which is not transcribed in normal H-$2^K$ lymphocytes, was transcribed in BW5147 cells.

To determine whether the $Q5^k$ gene of BW5147 codes for the immunologically defined Qa-2 antigen, analysis was carried out on reactivity of the enzyme-labelled antigen with the peptide encoded by the cDNA sequence amplified by PCR using primers No.5 and No.9.

The amplified DNA was ligated rightly into the SmaI site of pUC119 to obtain a recombinant, which was introduced into competent E. coli MV1184 cells. The fusion protein with lacZ protein was induced by

culturing the cells under the existence of IPTG.

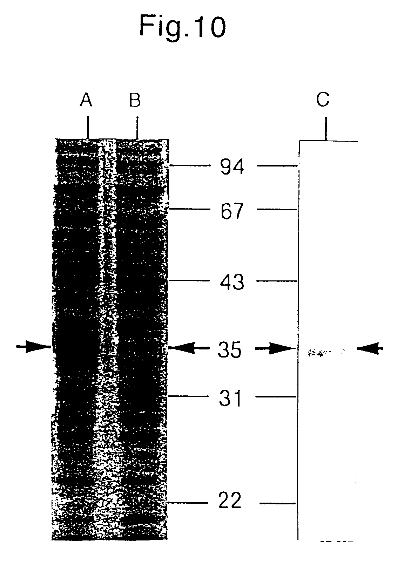

As shown in Fig. 10, the obtained E. coli showed the induced synthesis of a peptide with molecular mass of about 35 kDa, which was the value expected from the fusion protein. This 35 kDa protein reacted with peroxidase-labelled anti-Qa-2 mAb 59. The protein was not detected under identical conditions when peroxidase-labelled anti-Qa-2 mAb 34-1.2 was used.

From these results, the inventors concluded that the Qa-$2^k$ antigen on the surface of BW5147 cells, which was detected by mAb 59 but not by mAb 34-1.2, and which was resistant to treatment with PI-PLC, was the product of the Q$5^k$ gene.

Example 3:

From the experiments of the above Examples, it is evident that activation of Q$5^k$ gene in the Qa/T1a region is admitted, the Q5 gene product has common antigenicity (cross-reactivity) with normal Qa-2 lymphocyte antigen, and further, response of the Qa-$2^-$ mice bearing tumor cells against the Q5 gene product expressed by the tumor cells can target also the Qa-2 antigen (the product of Q7 gene) on the surface of the allogeneic normal lympocytes.

Therefore, the present inventors made experiments for inducing resistance against tumor cells by immunity using the Qa-2 antigen.

[method]

Qa-$2^-$ mice were immunized by normal lymphocytes of Qa-$2^+$ allogeneic mice. As Qa-$2^-$ mice, (C3H/He × B6.K1) $F_1$ mice were used, and administered intraperitoneally with splenocytes of C57BL/6 mice to immunize specifically to Qa-2 antigen. As tumor cells, Qa-$2^+$ tumor cells from C3H/He mice were used.

[Results]

(1) By the above immune treatment specific to Qa-2 antigen, resistance against tumor cells, such as MM2 of C3H/He mice origin, was induced in the $F_1$ mice. Namely, while transplantation of $2 \times 10^5$ tumor cells was effectuated in the control nontreated group, transplantation was not effectuated in the immunized mice.

Further, when the $F_1$ mice were immunized by lymphocytes of B6.K2 mice instead of C57BL/6 mice, same results were obtained as shown in the following Table 9.

Table 9

| mouse | rate of transplantation of MM2 cells |
|---|---|
| mouse immunized against Qa-2 antigen | 0/10* |
| Control untreated mouse | 10/10 |

* denominator: total number of mouse used
numerator : number of mouse to which transplantation was effectuated

(2) Splenocytes obtained from the immunized $F_1$ mice were stimulated in vitro by splenocytes of C57BL/6 mice again to induce T cells having cytotoxicity specific to Qa-2 antigen. These T cells stimulated strongly not only normal lymphocytes of Qa-$2^+$ allogeneic mice but also tumor cells from C3H/He mice.

Further, when splenocytes obtained from the $F_1$ mice immunized by lymphocytes of B6.K2 mice were stimulated in vitro by lymphocytes of B6.K2 mice, T cells having anti-Qa-2 cytotxicity were induced. Cytotoxic activities were measured against target tumor cells, and shown in the following Table 10.

Table 10

| Target cells | Cytotoxic activity (% specific lysis) |
|---|---|
| Tumor cells<br><br>MM2<br><br>Meth A | <br><br>$77.4 \pm 3.3$ %<br><br>$65.3 \pm 3.2$ |
| Controls<br><br>B6.K1 lymphocytes<br><br>B6.K2 lymphocytes | <br><br>$16.9 \pm 2.0$<br><br>$77.4 \pm 3.3$ |

Effector:Target = 100:1

(3) The $F_1$ mice were transplanted with NSFa tumor cells of C3H/He mice origin at their plantar portion. The mice were cut at their thighs to remove tumor cells when diameter of tumor reached 1 cm, and, 1 to 4 days thereafter, immunized specifically to Qa-2 antigen with splenocytes of C57BL/6 mice.

In all mice of the control untreated group, many lung metastases occured, and about 4 weeks after, all mice died of lung metastases, while no metastasis was observed in most mice although one or two metastases were found in a small number of mice.

Further, when the $F_1$ mice were transplanted with NFSa tumor cells derived from C3H/He mice at their plantar portion. The mice were cut at their thighs to remove tumor cells when diameter of tumor reached 8 to 10 mm, and, 3 days thereafter, immunized specifically to Qa-2 antigen by administering intraperitoneally splenocytcs of B6.K2 mice. After 17 days, mice were anatomized and their lungs were observed under a stereoscopic microscope to count lung metastases to obtain a following Table 11.

Table 11

| Group | number of lymphocytes used for immunizing | number of lung metastases | | | |
|---|---|---|---|---|---|
| | | C3H/He × B6.K1 F$_1$ mice | | C3H/He × B6.K2 F$_1$ mice | |
| | | mouse | number of nidi | mouse | number of nidi |
| 1 | $5 \times 10^6$ | No.1 | 0 | No.1 | 20 |
| | | No.2 | 0 | No.2 | 6 |
| | | No.3 | 0 | No.3 | 11 |
| | | No.4 | 0 | | |
| 2 | $5 \times 10^5$ | No.1 | 0 | No.1 | 9 |
| | | No.2 | 1 | No.2 | 8 |
| | | No.3 | 0 | No.3 | 10 |
| | | No.4 | 1 | | |
| 3 | $5 \times 10^4$ | No.1 | 5 | No.1 | 6 |
| | | No.2 | 8 | No.2 | 12 |
| | | No.3 | 6 | No.3 | 14 |
| 4 | Control group with no immunization | No.1 | 5 | No.1 | 9 |
| | | No.2 | 9 | No.2 | 14 |
| | | No.3 | 9 | No.3 | 10 |

Thus, it is evident that registance against tumor cells can be induced by immunizing Qa-2⁻ with Qa-2⁺ allogeneic normal lymphocytes. Namely, immunoreaction against the Q7 gene product in Qa-2⁻ mice has cross-reactivity with Q5 gene product, and it is evident that this cross-reactivity forms the above registance against tumor cells.

Example 4:

As to non-classical histocompatibility class 1 antigens of human being, in some of the antigens, all nucleotide sequence of the gene has been decided already. It is known also that some of gene are inactive in normal adults.

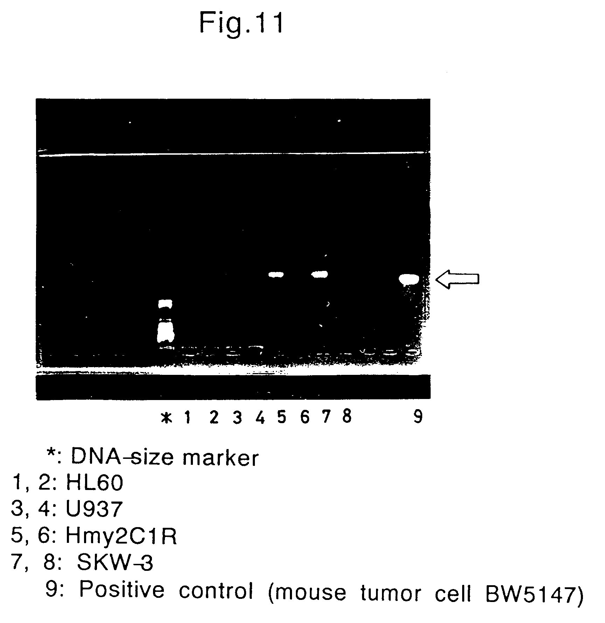

Then, a polynucleotide, which has a nucleotide sequence specific to such a gene, is prepared and used as a primer in trying to amplify cDNAs prepared from human tumor cell strains (HL60, U937, Hmy2C1R and SKW-3) by PCR method. As shown in Fig. 11, nucleotide amplifications of expected length (shown by arrow) were detected.

This result shows that non-classical histocompatibility class 1 antigens, which are not activated in normal adult, were activated in human tumor cells.

As described above, registance against tumor cells is induced in Qa-2⁻ mice by immunizing the Qa-2⁻ mice with Qa-2⁺ allogeneic normal lymphocytes. Further, in tumor cells of Qa-2⁺ mice origin, Q5 gene, which is not activated in normal adult mice, is activated, and therefore, immunoreaction specific to Q5 gene product can be induced by immune treatment using Q5 gene product, thus making it possible to induce registance against autogenetic tumor cells.

Moreover, in human beings also, non-classical histocompatibility class 1 antigens, which are not activated in normal adults, are activated in tumor cells.

Accordingly, an anti-tumor method and an anti-tumor agent are provide by the present invention. Namely, it is possible to induce registance against tumor cells by administering a non-classical histocompatibility class 1 antigen, an antibody which identifies that antigen, or a cytotoxic lymphocyte.

**Claims**

1. An anti-tumor method comprising administering a non-classical histocompatibility class 1 antigen.

2. An anti-tumor method comprising administering an antibody which identifies a non-classical histocompatibility class 1 antigen.

3.  An anti-tumor method comprising administering a cytotoxic lymphocyte which identifies a non-classical histocompatibility class 1 antigen.

4.  An anti-tumor agent comprising a non-classical histocompatibility class 1 antigen as an effective component.

5.  An anti-tumor agent comprising, as an effective component, an antibody which identifies a non-classical histocompatibility class 1 antigen.

6.  An anti-tumor agent comprising, as an effective component, a cytotoxic lymphocyte which identifies a non-classical histocompatibility class 1 antigen.

# Fig.1

( ———— ) : Qa-2-specific mAb 141-15.8
( - - - - - ) : mAb specific to a bacterial component (negative control)

# Fig.2

● : anti-Qa-2 mAb
■ : FPLC-purified material

% Specific lysis vs. Protein concentration ( μg / incubation)

# Fig.3

A

(kDa)   a   b

200.0 —

116.5 —

97.4 —

66.2 —

B

acidic                    basic

(kDa)

— 97.4

— 66.2

— 42.9

— 31.0

— 21.5

# Fig.4

EP 0 563 627 A2

Fig.5

## Fig.6

| Exon 1 | Exon 2 | Exon 3 | Exon 4 | Exon 5 | Exon 6 | Exon 7 | Exon 8 |
|--------|--------|--------|--------|--------|--------|--------|--------|

NO.1 →

NO.2 →

NO.3 →

NO.4 →

NO.5 →

NO.6 →

NO.7 →

← NO.8

NO.9 →

← NO.11

← NO.10

# Fig.7

## Fig.8

# Fig.9

# Fig.10

# Fig.11

* 1 2 3 4 5 6 7 8   9

  *: DNA–size marker
1, 2: HL60
3, 4: U937
5, 6: Hmy2C1R
7, 8: SKW–3
  9: Positive control (mouse tumor cell BW5147)